# EUROPEAN PATENT SPECIFICATION

(11) **EP 1 911 484 B1**
(45) Date of publication and mention of the grant of the patent: **22.09.2010**
(21) Application number: 08001306.3
(22) Date of filing: 10.12.2001
(51) Int. Cl.: A61M 25/00, A61B 5/0215, A61M 25/10

(54) **Intra-aortic balloon catheter having a fiberoptic sensor**
Intraaortaler Ballonkatheter mit Glasfasersensor
Cathéter ballonnet intra-aortique disposant d'un capteur fibro-optique

(30) Priority: 12.12.2000 US 734755; 09.08.2001 US 925143
(43) Date of publication of application: 16.04.2008
(62) Divisional of application: 01270357.5
(73) Proprietor: DATASCOPE INVESTMENT CORP., Montvale, N.J. 07645 (US)
(72) Inventor: Shock, Robert B., Sparta NJ 07871 (US); Williams, Jonathan, Montville NJ 07045 (US); Walters, Daniel A., Rockaway Township NJ 07801-1918 (US)
(74) Representative: Schmidt, Karsten

(56) References cited:
- EP-A- 0 397 055
- EP-A- 0 611 582
- EP-A- 0 778 037
- WO-A-99/17831
- US-A- 4 924 877
- US-A- 5 209 727
- US-A- 5 313 957
- US-A- 5 427 114
- US-A- 5 429 597
- US-A- 5 701 905
- US-A- 5 928 155
- TOHYAMA O ET AL: "A fiber-optic pressure microsensor for biomedical applications" 1 April 1998 (1998-04-01), SENSORS AND ACTUATORS A, ELSEVIER SEQUOIA S.A., LAUSANNE, CH, PAGE(S) 150 - 154 , XP004143984 ISSN: 0924-4247 * the whole document *

## Description

### BACKGROUND OF THE INVENTION

### Field of the Invention

The invention relates to a catheter having enhanced pressure sensing capabilities. More particularly, the invention relates to single and dual lumen intra-aortic balloon catheters having a fiberoptic sensor connected to the catheter.

### Description of the Prior Art

A key function of many catheters is that of continuously monitoring blood pressure. In many cases, this monitoring must be performed with accurate measurement of high frequency components. For example, reliable detection of the dicrotic notch of the aortic blood pressure waveform typically requires a pressure signal having a bandwidth of 15Hz or better. Detection of the dicrotic notch is generally used for the inflation/deflation timing of an intra-aortic balloon ("IAB") catheter.

Conventional invasive pressure monitoring is performed with low-cost fluid-filled transducers. A typical disposable monitoring kit, inclusive of all tubing, a continuous flush device, and a pre-calibrated transducer is very affordable. Unfortunately, these systems have several drawbacks. One major drawback is that bubbles or clots in the monitoring lines can reduce the frequency response of the system to a level below 15 Hz, creating an "overdamped" condition. In other cases, the characteristics of the catheter and tubing can result in "ringing", which is associated with an underdamped condition. Furthermore, fluid-filled catheters can suffer from "catheter whip" (motion artifact), which is manifested as one or more high frequency deflections in the pressure signal. These problems can degrade the usefulness of the signal in applications such as intra-aortic balloon pumping (IABP). In particular, it is difficult, if not impossible, to automatically provide optimal timing of IABP using a pressure signal with a frequency response below 15Hz, or using signals with ringing or whip artifacts that mimic the physiologic dicrotic notch.

Another disadvantage of the traditional fluid-filled transducer is that it requires the use of an inner guidewire tube. Traditionally, IAB catheters have two lumens in the catheter: a gas shuttle lumen and an inner guidewire lumen. The gas shuttle lumen has to be large enough to allow the gas to shuttle back and forth without undue restriction to ensure speedy inflation and deflation of the IAB membrane. In order to make the catheter smaller it is desirable to remove the inner guidewire lumen or tube, thus requiring an alternate means for measuring arterial pressure. EP 0611582 A2 describes an Intra-aortic balloon catheter for placement using an indwelling guide wire having guide structures which reduce the size of the catheter, which can increase distal perfusion and facilitate insertion and placement. These guide structures include short internal or external lumens, formed from separate guide sleeves or by method of wrapping the balloon membrane itself. The guide sleeves can be located on or inside the balloon membrane or can be attached to the balloon tip or catheter tube, and can be permanent or detachable.

Another means for monitoring blood pressure is to use a micromanometer, such as marketed by companies such as Millar, Endosonics, and Radi. See U.S. Patents Nos. 5,431,628 and 5,902,248. These devices can have excellent frequency responses, with system bandwidths greater that 200Hz. They are not subject to the negative effects of bubbles and catheter whip, and retain good performance even in the presence of small blood clots. Unfortunately, they are very expensive, prone to signal drift, and can suffer from electrical interference. A common source of electrical interference in the setting of IABP therapy is the use of electrosurgery. In this situation, it is desirable to maintain a reliable pressure signal with which to trigger the balloon, as the ECG signal which normally triggers IABP operation become∼ completely unreliable. Conventional fluid-filled transducer systems are relatively immune from this type of interference. Attempts have been made to use micromanometers for IABP timing, see U.S. Patent Nos. 3,585,983 and 4,733,652. These attempts proved to be unreliable, as the device may be damaged during insertion and is also prone to signal drift. To address the drift issue, U.S. Patent no. 5,158,529 discloses a method for rezeroing the micromanometer by using the pressure from a partially filled balloon as it rests in the aorta. However, this method requires momentary interruption of IABP, which may be harmful to the critically ill patient.

While standard IAB catheters incorporating a fluid-filled transducer pressure measurement system or IAB catheters incorporating micromanometers may be suitable for the particular purpose employed, or for general use, they would not be as suitable for the purposes of the present invention as disclosed hereafter.

### SUMMARY OF THE INVENTION

Accordingly, there is a need for a reliable and affordable pressure monitoring approach that has high bandwidth pressure sensing, low signal drift, and freedom from electrosurgical interference. There is also a need to incorporate this technology into intra-aortic balloon catheters having small cross sectional profiles.

The invention is an IAB catheter system as defined in independent claim 1, with either a double or single lumen, having enhanced blood pressure sensing capability. The IAB catheter has a fiberoptic sensor built into the tip of the IAB or connected to another part of the catheter.

Fiberoptic sensors have the advantage of being immune to electrical interference, and have the potential to be lower in cost than electronic micromanometers.

To the accomplishment of the above and related objects the invention may be embodied in the form illustrated in the accompanying drawings. Attention is called to the fact, however, that the drawings are illustrative only. Variations are contemplated as being part of the invention, limited only by the scope of the claims.

### BRIEF DESCRIPTION OF THE DRAWINGS

In the drawings, like elements are depicted by like reference numerals. The drawings are briefly described as follows.

FIG 1 is a longitudinal cross sectional view of a distal end of a coaxial IAB catheter having a fiberoptic sensor in the tip.

FIG 1A is a perspective view of distal end of inner tube 58, shown independent of catheter 10, with pressure sensing line 24 connected to an outer surface of inner tube 58.

FIG 1B is a perspective view of a distal end of inner tube 58, shown independent of catheter 10, with pressure sensing line sandwiched between an outer surface of inner tube 58 and an outer layer.

FIG 1C is a perspective view of a distal end of inner tube 58, shown independent of catheter 10, with pressure sensing line 24 embedded in the wall of inner tube 58.

FIG 2 is a longitudinal cross sectional view of a distal end of a co-lumen IAB catheter having a fiberoptic sensor in the tip.

FIG 2A is a transverse cross sectional view of the co-lumen IAB catheter in FIG 2 taken along lines 2A-2A.

FIG 3A is a longitudinal cross sectional view of the tip having an embedded fiberoptic sensor in a first configuration.

FIG 3B is a longitudinal cross sectional view of the tip having an embedded fiberoptic sensor in a second configuration.

FIG 3C is a longitudinal cross sectional view of the tip having an embedded fiberoptic sensor in a third configuration.

FIG 3D is a longitudinal cross sectional view of the tip having an embedded fiberoptic sensor in a fourth configuration.

FIG 4 is a longitudinal cross sectional view of a single lumen IAB catheter having a fiber optic sensor in the tip.

FIG 5A is a perspective view of the single lumen catheter of FIG 2 having a monorail tip and a guide wire passing through it.

FIG 5B is a perspective view of the single lumen catheter of FIG 2 having a monorail tip and a guide wire passing through it as well as the balloon membrane.

FIG 6 is a perspective view of a distal end of the catheter connected to the stylet via a catheter tongue extension.

FIG 7A is a perspective view of a transparent wire reinforced catheter having a straight exposed distal portion forming a stylet.

FIG 7B is a perspective view of a transparent wire reinforced catheter having an exposed straight distal wire portion connected to a tube to form a stylet.

FIG 7C is a perspective view of a transparent wire reinforced catheter having an exposed helical distal wire portion connected to a tube to form a stylet.

### DETAILED DESCRIPTION OF THE PREFERRED EMBODIMENTS

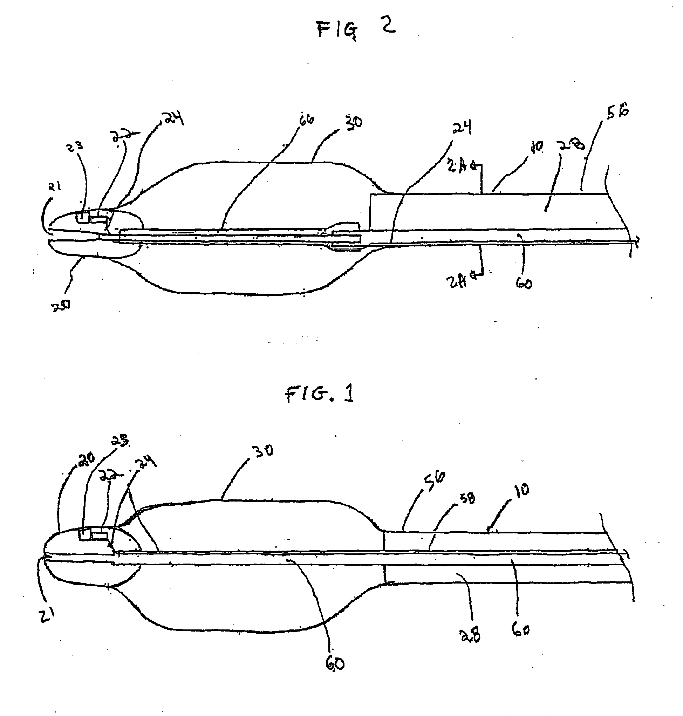

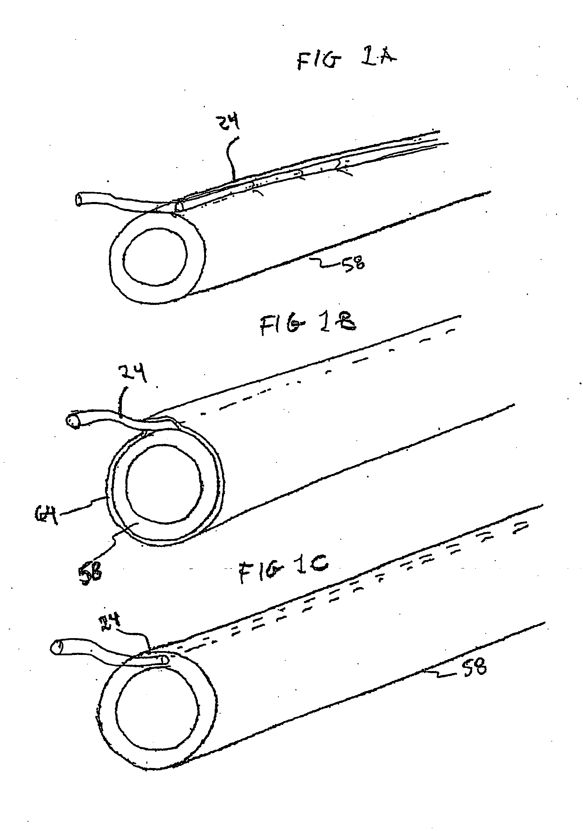

FIG 1 illustrates a longitudinal cross section of a distal portion of a dual lumen intra-aortic balloon ("IAB") catheter 10 comprising an outer tube 56, an inner tube 58, a tip 20, and a balloon membrane 30 connected on one end to the outer tube 56 and on the opposite end to the tip 20. Tip 20 defines a tip lumen 21. Inner tube 58 is disposed within the outer tube 56 and is connected to the tip 20 at its distal end. Inner tube 58 defines an inner lumen 60 and outer tube 56 defines an outer lumen 28. Outer lumen 28 is used for shuttling helium or another appropriate working gas or fluid for inflation and deflation of the balloon membrane 30. Outer tube 56 may be coil or braid reinforced and made from polyurethane or polyimide. Inner tube 58 may be made from polyimide or an alloy with shape memory and superelastic properties commonly referred to as Ni-Ti, NITINOL™, and other industry names. Inner tube 58 may be connected to an inner surface of the outer tube 56 at one or more points to enhance pushability, stability, pumping speed, and pressure fidelity. Furthermore, inner tube 58 may be made from two or more tubes joined end-to-end, as disclosed in U.S. Patent No. 6,024,693, herein incorporated by reference in its entirety, or may have an inner tube having a variable diameter, as disclosed in U.S. Patent Application No. 09/764831, filed on January 17, 2001. Fiber optic sensor 22 is embedded in or attached to the tip 20 and preferably has a diameter less than or equal to 0.762 mm (0.03 inches). A forward looking end of the fiberoptic sensor 22 faces chamber 23. Pressure sensing line 24 may be affixed to inner tube 58 and connects fiber optic sensor 22 to an optoelectronic interface (not shown) via an industry-standard "SC" connector (not shown). Pressure sensing line 24 preferably is made of glass, but may be made of plastic or another material, and preferably has a diameter less than or equal to 0.152 mm (0.006 inches). Note that fiberoptic sensor 22 may be positioned in alternate locations along IAB catheter 10 as well as on a distal tip of an independent catheter that can be disposed within inner lumen 58.

FIGS 1A-1C illustrate perspective views of a distal end of inner tube 58 with pressure sensing line 24 connected to inner tube 58 in various configurations. In FIG 1A, pressure sensing line 24, is connected to an outer surface of inner tube 58. In FIG 1B, pressure sensing line 24 is disposed between inner tube 58 and a thin walled tube 64, which preferably is heat shrinkable. Heat shrinkable tubing with a wall thickness of less than or equal to 0.0254 mm (0.001 inches) is available from Advanced Polymers, Inc., Salem, NH. In FIG 1C, pressure sensing line 24 is embedded in the wall of inner tube 58. Note that although pressure sensing line 24 is shown running along a longitudinal axis of inner tube 58 it may also be wound helically. Embodiments shown in Fig. 1A-1C are not specifically claimed.

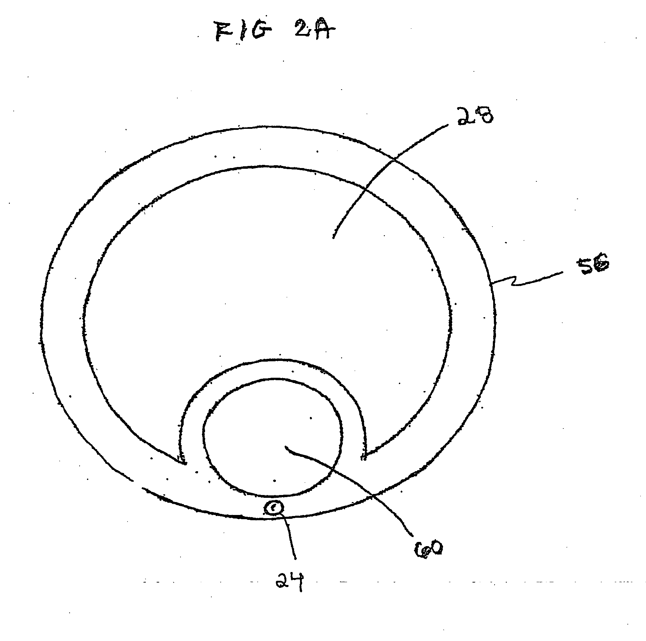

FIG 2 illustrates a distal portion of another embodiment of the IAB catheter 10, comprising a balloon membrane 30, a tip 20, a co-lumen tube 56, an inner lumen extension tube 66, and a fiberoptic sensor 22. Detailed structure of a co-lumen IAB is disclosed in U.S. Patent No. 6,024,693 and U.S. Patent Application Serial No.09/412,718, filed on October 5, 1999. Tip 20 is connected to a distal end of the balloon membrane 30 and to a distal end of the inner lumen extension tube 66. Tip 20 defines a tip lumen 21. A distal end of the co-lumen tube 56 is connected to a proximal end of the balloon membrane 30 and to a proximal end of the inner lumen extension tube 66. The co-lumen tube 56 may be coil or braid reinforced and made from polyurethane or polyimide. The preferred material for inner lumen extension tube 66 is an alloy with shape memory and superelastic properties commonly referred to as Ni-Ti, NITINOL™, and other industry names. Inner lumen extension tube 66 may also be made from polyimide. The fiberoptic sensor 22 is attached to tip 20 and pressure sensing line 24 which communicates signals generated by the fiberoptic sensor 22 to an optoelectronic interface (not shown). The pressure sensing line 24 may be connected to the inner lumen extension tube 66 and co-lumen tube 56 in any of the ways pressure sensing line 24 is connected to the inner tube 58, illustrated in FIGS 1A-1C. Furthermore, the pressure sensing line may be embedded in the outer tube 56 as illustrated in FIG 2A.

FIG 2A illustrates a transverse cross section of outer tube 56, taken along line 2A-2A illustrated in FIG 2, with pressure sensing line 24 embedded in the wall. Outer tube 56 defines two distinct lumens, inner lumen 60 and outer lumen 28. Outer lumen 28 is used for shuttling helium or another appropriate fluid or gas for inflation and deflation of balloon membrane 30. Note that pressure sensing line 24 may be embedded at a different location in outer tube 56, may be connected to a surface of outer tube 56, or may freely reside in outer lumen 28. Note also that the fiberoptic sensor 22 may be positioned in alternate locations along IAB catheter 10 as well as on a distal tip of an independent catheter that can be disposed within the inner lumen 60. Embodiments shown in Fig. 2A are not specifically claimed.

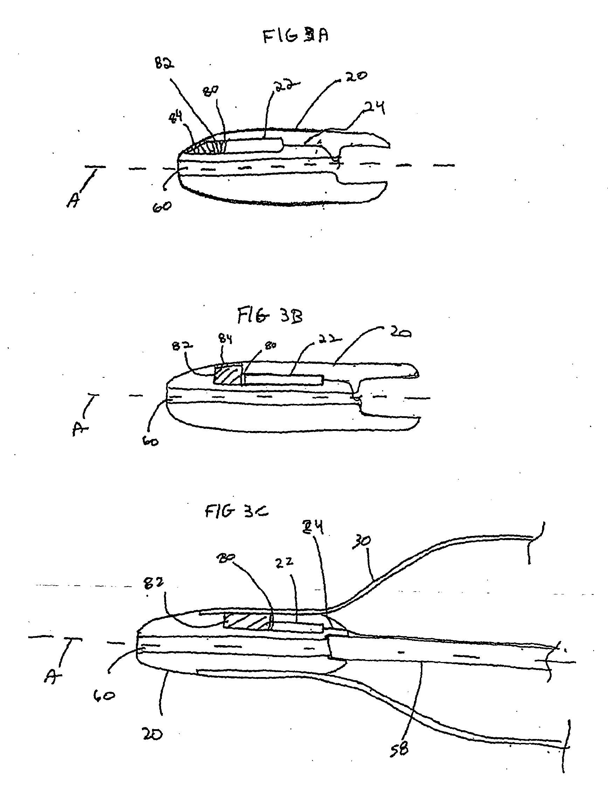

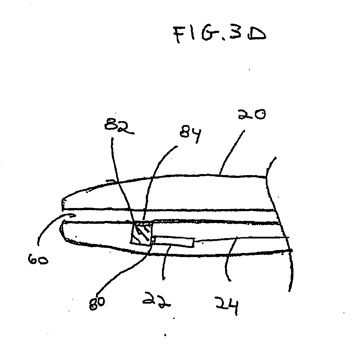

FIGS 3A-3D illustrate alternate configurations for tip 20 incorporating a forward looking fiber optic sensor 22. In FIG 3A and FIG 3B longitudinal cross sections of tip 20 are shown independent of the rest of catheter 10 for clarity. In FIG 3A fiber optic sensor is embedded in tip 20 parallel to the longitudinal axis of tip 20 labeled A. Fiber optic sensor 22 has an approximate outer diameter of 0.0635 mm (0.0025 inches) and has a pressure sensing surface or diaphragm 80 on a distal end which is exposed to protective pocket 82. Diaphragm 80 may be made from silicone approximately 6 microns thick and is protected from physical damage and blood contact by protective pocket 82, which may contain a gel, fluid, gas, elastomer, or any other flexible protective material. Diaphragm 80 may project into the pocket or may be flush with the protective pocket 82 wall. Membrane 84 prevents leakage of gel or protective pocket 82. Pressure sensing line 24 comprises a fiberoptic fiber having an outer diameter of approximately 0.152 mm (0.006 inches). In FIG 3B (and also FIGS 1 and 3) the gel or protective pocket 82 is shifted proximally. In FIG 3C a distal portion of balloon membrane 30 is used to seal protective pocket 82. In FIG 3D protective pocket 82 opens into inner lumen 60. Membrane 84 prevents leakage of gel out of protective pocket 82. In this location fiberoptic sensor 22 is exposed to arterial pressure via tip lumen 21 and is less likely to be damaged upon insertion and placement of IAB catheter 10. Note that although forward looking fiber optic pressure sensors are illustrated, use of side looking sensors is anticipated as well.

In addition to sensing blood pressure, the fiberoptic sensor 22 can be used to measure other important physiologic variables, such as, but not limited to temperature (as in the Luna Innovations sensor noted below), pCO2, pO2, pH, oxygen saturation, lactate, CPK, anesthetic agent concentration, various biological compounds, electrolytes, and nitric oxide. Blood pressure, as well as at least some of these variables, could be measured using either extrinsic or intrinsic fiberoptic sensors. Extrinsic sensors use the fiberoptic fiber to communicate information from a separate structure which is attached to the fiber. Intrinsic sensors sense information as a result of physical changes of the fiber itself. Intrinsic sensors have a particular advantage of being extremely small, rugged, and low in cost.

As indicated above the pressure sensing line 24 terminates in an optoelectronic interface which provides a light source, light detector, optical components (such as a lens, splitter, and interferometer), power supply, and signal processing means. This optoelectronic interface is known in the art and can be connected to or integrated with another electronic device, such as a monitor or intra-aortic balloon pump. Light originating from the interface travels down the fiberoptic fiber, i.e. the pressure sensing line 24, by the well-known principal of total internal reflection to the sensing element. The sensing element incorporates pressure responsive means which modifies the character of the light as a function of pressure. The modified light is in turn reflected back through the fiber to the interface. The optical components, light detector, and signal processing elements of the interface work together to produce an electrical output that characterizes the pressure at the sensor. This output is translated into a displayed pressure waveform or digital pressure display by the IABP or monitor.

Fiberoptic pressure monitoring sensors and optoelectronic interfaces suitable for this application are manufactured by several companies, including FISO Technologies Inc. (Quebec, Canada) and Luna Innovations (Blacksburg, VA). FISO utilizes a white light interferometric approach, as described in U.S. patents nos. 5,202,939 and 5,392,117. Luna Innovations uses a laser light source in an approach similar to that described in U.S. patent no. 5,301,001.

There are a variety of possible configurations for the fiberoptic sensor itself. One type would employ a deformable diaphragm separated from the end of one or more fiberoptic fibers by a chamber. The chamber may be filled with air and vented to atmosphere to eliminate temperature-induced pressure changes within the chamber (these would be associated with signal drift). A disadvantage of this design in an IAB catheter is that an air-venting tube is required which occupies a portion of the cross-sectional area of the catheter. This tube reduces the potential to produce the catheter in a small size with acceptable performance.

A more desirable approach to a fiberoptic pressure sensor for a small catheter is to utilize a design without a vent tube (as illustrated in FIGS 1, 2, and 3A-3D). Such a sensor can be produced by positioning the sensor diaphragm over a chamber with a vacuum (as currently practiced by FISO). With this approach, a sensor can be constructed which has little temperature-induced drift, assuming that the materials which are used to build the diaphragm and chamber have small coefficients of thermal expansion (ceramics, glass, and silicon are good choices). This approach makes the device an absolute pressure sensor, which requires a separate barometric pressure sensor to convert absolute pressures to gauge pressures. In IABP applications, such a barometric pressure sensor could be built into the pump. U.S. Patent No. 5,158,529, teaches another possible means for assuring the accuracy of gauge pressure values as indicated by a sensor in the IAB catheter; this approach compares the blood pressure values indicated by the catheter sensor with pressure values in the gas within a partially-filled catheter and pump, and adjusts the sensor-indicated pressure to agree with the gas pressure.

Another approach to constructing a non-vented pressure sensor is to trap a gas in the chamber beneath the diaphragm and to monitor its temperature (as currently practiced by Luna Innovations). In this case, knowledge of the gas temperature within the chamber allows for correction of temperature-induced pressure changes. Ideally, if a gas is to occupy the chamber, it is dry and inert. It is possible that appropriate selection of the bias gas would improve linearization of the sensor or change its dynamic range. Embodiments shown in Fig. 3A-3D are not specifically claimed.

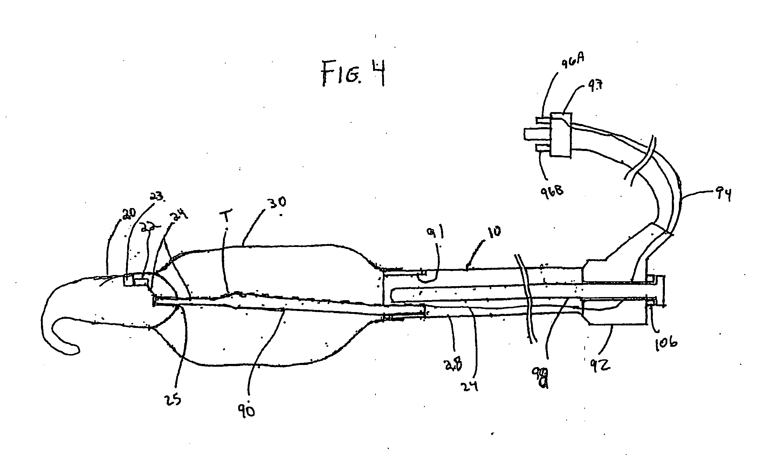

FIG 4 illustrates a single lumen IAB catheter 10, comprising balloon membrane 30, a J-shaped tip 20, a stylet 90, a gas lumen insert 98, and a fiberoptic sensor 22. Tip 20 is connected to a distal end of balloon membrane 30 and preferably has an outer diameter of approximately 6 millimeters. Balloon membrane 30 preferably has a single wall thickness of approximately 0.0584 mm (0.0023 inches) and defines a volume of 40 cubic centimeters. Fitting 92 is connected to a proximal end of catheter 10. Tube 94 connects connector 96 to fitting 92. Fiber optic sensor 22 is embedded in or attached to tip 20 and preferably has a diameter less than or equal to 0.762 mm (0.03 inches) A forward looking end of fiberoptic sensor 22 faces chamber 23. Pressure sensing line 24 may be free-floating within a lumen in catheter 10 or embedded in catheter 10 similar to the connection configurations illustrated in FIGS 1A-1C in connection with inner tube 58. Pressure sensing line 24 connects fiber optic sensor 22 to an optoelectronic interface (not shown) via an industry-standard "SC" connector 96A.

In addition to connector 96A, connector hub 97 may also include data input/out connector 96B for data input and output beneficial to optimization of the device and/or data logging onto a data storage device incorporated into connector hub 97 (not shown). Incorporation of connector 96B results in a fully integrated pneumatic, electrical, and electro-optical connector hub.

A distal end of stylet 90 is connected to tip 20 inside tip lumen 25. A proximal end of stylet 90 is connected to an inner surface of a reinforcement ring or skirt 91 which in turn is connected to an inner surface of a distal end of catheter 10. Skirt 91 is preferably made from stainless steel but can also be made from Nitinol or another appropriate stiff material.

Pressure sensing line 24 preferably is made of glass, but may be made of plastic or another material, and preferably has a diameter less than or equal to 0.152 mm (0.006 inches). Pressure sensing line 24 is connected to an outer surface of stylet 90 but may also be connected in any of the ways illustrated in FIGS 1A-1C. If stylet 90 is made from a tube, pressure sensing line 24 may pass through the tube. Pressure sensing line 24 is disposed within catheter 10 and passes through fitting 92, tube 94, and terminating in connector 96.

Note that all the details regarding pressure sensor 22 detailed above for the coaxial and co-lumen catheters apply equally to the single lumen embodiment. Note further that pressure sensor 22 for the single lumen embodiment is optional. Pressure information may be obtained through other means, such as but not limited to, pressure lines placed elsewhere on the patient's body, e.g. radial line. Another alternative would be to employ an alternative timing signal, such as blood flow or heart sounds.

Catheter 10 is preferably made from a spiral wire reinforced polyurethane, polyimide, or Pebax (Pebax is a trademark of Elf Atochem Inc. of Birdsboro, PA) material. The outer diameter of catheter 10 is preferably 2.16 mm (0.085 inches) and the inner diameter is preferably 1.85 mm (0.073 inches). Note that the preferred dimensions translate to approximately a 6 Fr. catheter (a one millimeter diameter is approximately 3 Fr.) which is a significant size reduction compared to current dual lumen catheters on the market the smallest of which is an 8 Fr. catheter, sold by Datascope Corp.

The lack of a guidewire lumen allows for a significant reduction in the outer diameter of catheter 10, roughly 2 Fr. Given the fact that catheter 10 no longer has a guidewire lumen the catheter design must allow for insertion without a guidewire. This is accomplished through the incorporation of stylet 90, removable gas lumen insert 98, and J-shaped tip 20 into the design of catheter 10.

Removable gas lumen insert 98 is removably disposed within catheter 10 and connects to fitting 92 via a connector 106. As detailed in U.S. Patent Application No. 09/813,905, filed on March 21, 2001, catheter 10 is inserted into a patient with gas lumen insert 98 disposed within catheter 10 to enhance the stiffness of catheter 10, and thus, facilitate insertion. After insertion gas lumen insert 98 is removed, vacating lumen 28, and allowing for shuttling of gas through lumen 28 for inflation and deflation of balloon membrane 30. Gas lumen insert 98 is preferably made from stainless steel but may also be made from Nitinol or another appropriate stiff material.

Stylet 90 is preferably made from solid stainless steel or Nitinol wire having a 0.508 mm (0.020 inch) diameter tapering down at point T towards tip 20 to a 0.254 mm (0.010 inch) diameter. Stylet 90 may also be made from a tube and from any other material having the appropriate stiffness necessary for IAB insertion and therapy. Alternate means for incorporating stylet 90 into IAB catheter 10 are disclosed in U.S. Patent No. 5,716,373, issued Wolvek et al.. The graduated diameter, and thus, stiffness of stylet 90 allows IAB catheter 10 to mimic the ability of a typical guidewire to pass through tortuous arteries. Note that location of the transition point T may vary, as well as the total variation of diameter and the degree of transition, so long as the distal end of the stylet is less stiff than the proximal end. Furthermore, to accomplish the transition in stiffness two tubes having varying diameters or different material properties may be connected end-to-end. Alternatively, a single tube having varying material properties, i.e. stiffness, along its length may be used.

As a first step in the insertion process, a standard guidewire is placed into a patient's blood vessel. If a sheath is to be used, a sheath-dilator assembly is then advanced over the guidewire in a standard manner. To maximize the ease of insertion a long sheath, approximately 279 mm (11 inches) to 381 mm (15 inches), may be used. The dilator and guide wire are then removed and the IAB catheter 10 is advanced into the sheath until it reaches the desired position in the aorta. Prior to the initiation of therapy gas lumen insert 98 is unlocked from connector 106 and pulled out from catheter 10. After removal of stylet 90 the port through which it was positioned in fitting 92 is sealed by a means (not shown) such as a cap, a self-sealing valve, or a manually sealable valve.

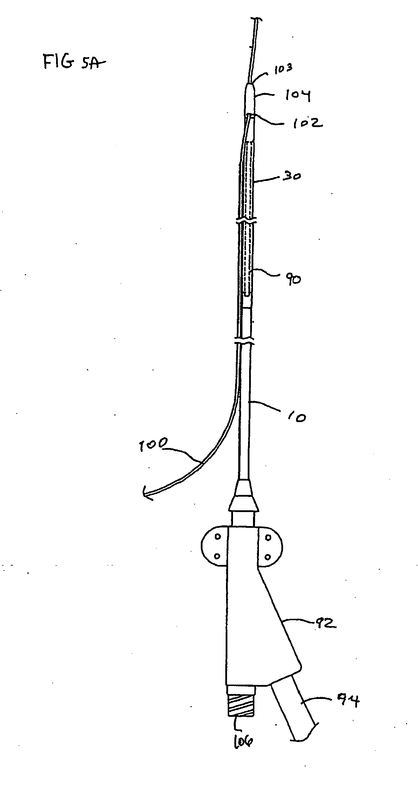

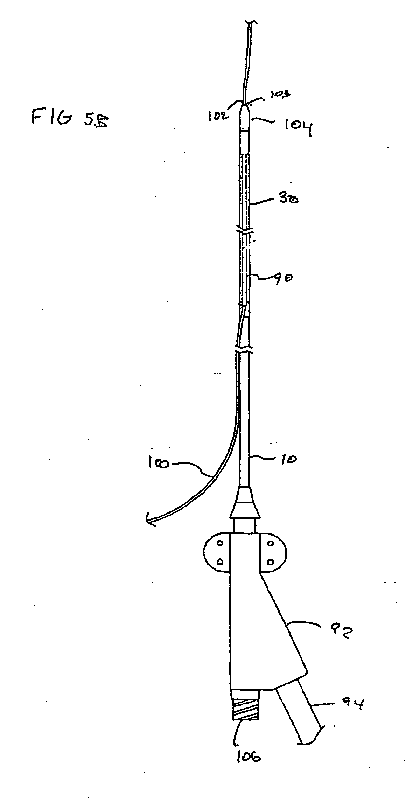

In an alternative embodiment, as illustrated in FIGS 5A and 5B, catheter 10 may have a monorail tip which allows for a sheathless insertion. FIG 5A illustrates a perspective view of catheter 10 with a guide wire 100 disposed within a lumen 102 in monorail tip 104. Stylet 90 is revealed using shadow lines. Balloon membrane 30 is wrapped around stylet 90 to minimize the cross section profile of the system for insertion. Preferably the outer diameter of the wrapped balloon membrane 30 is equal to or less than an outer diameter of catheter 10, as disclosed in U.S. Patent Application Serial No. 09/210,922, filed on December 14, 1998. Guidewire 100 enters tip 104 through a distal opening 103 and exits through a proximal opening. This is in contrast to the embodiment illustrated in FIG 5B where guidewire 100 enters through distal opening 103 in tip 104 but then continues through balloon membrane 30 and finally exits through an opening in catheter 10 near the proximal end of balloon membrane 30.

In the case of a sheathless insertion, guidewire 100 is first advanced into the aorta as described above and a proximal end of guidewire 100 is inserted through lumen 102 within tip 104. Flexible J-shaped tip 104 is straightened by guide wire 100. IAB catheter 10 is then advanced into the sheath until it reaches the desired position in the aorta. Next, guidewire 100 is removed, allowing tip 104 to recover its J-shape. Finally, gas lumen insert 98 is pulled out of catheter 10 and therapy can be initiated.

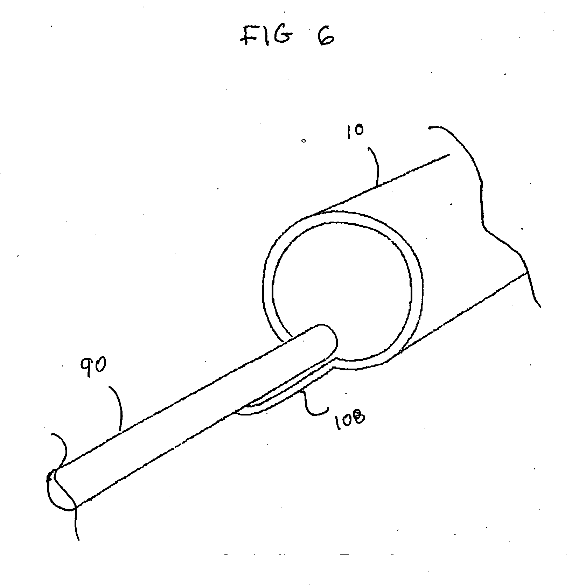

An alternate approach to bonding sylet 90 to catheter 10 is illustrated in FIG 6. Stylet 90 is fixed to a tongue 108 extending from a distal end of catheter 10 using any one of a variety of bonding methods, including but not limited to, a crimped sleeve, heat-shrink tubing, adhesive, or radiofrequency induced melt bonding. A proximal portion of stylet 90 may be roughened or contoured to optimize strength of the joint.
With the use of reinforced tubing for catheter 10 several other unique attachment methods for stylet 90 are possible.

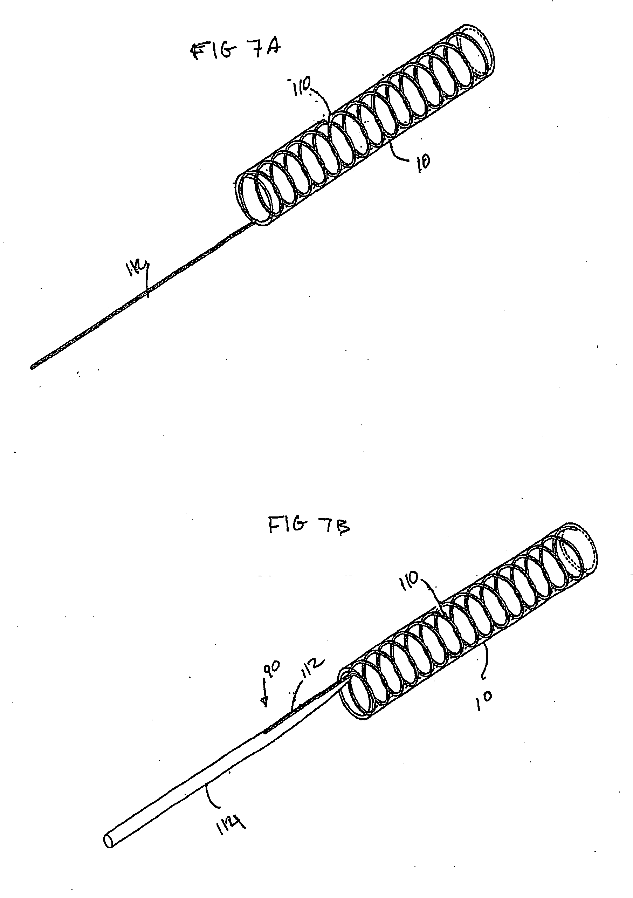

With the use of reinforced tubing for catheter 10 several other unique attachment methods for stylet 90 are possible. FIG 7A illustrates a perspective view of transparent catheter 10 having a wire reinforcement 110. A distal unwound portion 112 of wire reinforcement 110 serves as a stylet. If a distal portion of wire reinforcement 110 is not stiff enough it may be joined to a tube 114 or a secondary wire having the desired properties, including the transition in diameter and/or stiffness, as illustrated in FIG 7B. Note that a proximal portion of tube 114 is optionally angled so as to control the stiffness of the wire/tube joint.

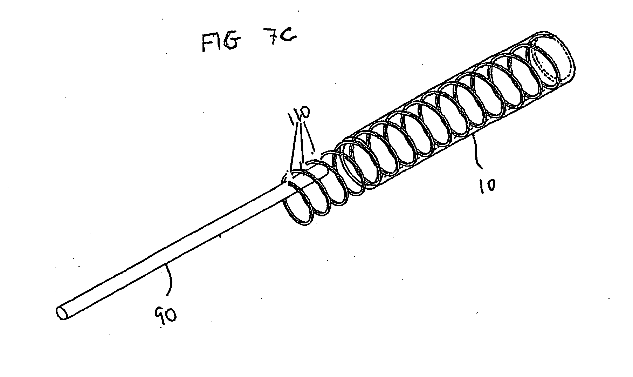

FIG 7C illustrates a third method for joining stylet 90 to catheter 10. In this case, stylet 90 is fixed to a distal portion of exposed wire reinforcement 110. Optionally, there is an exposed portion of catheter 10 that is not joined to stylet 90. This un-joined exposed portion acts as a joint of specific flexibility for optimization of the stylet-to-catheter joint and allows for a better transition between the mechanical characteristics of stylet 90 and catheter 10.

As many apparently widely different embodiments of the present invention can be made without departing from the scope thereof, it is to be understood that the inventio is not limited to the specific embodiments thereof except as defined in the appended claims. Embodiments shown in Fig. 5A-7C are not specifically claimed.

## Claims

1. A balloon catheter (10) comprising a balloon membrane (30), a tube (56), a tip (20), and a stylet (90), a distal end of the tube is connected to a proximal end of the balloon membrane, the tip is connected to a distal end of the balloon membrane, a distal end of the stylet is connected to the tip, a proximal end of the stylet is connected to a distal end of the tube, the stylet being more flexible towards the distal end than the proximal end, wherein a reinforcement ring (91) is disposed within a distal end of the tube (56), **characterized in that** a proximal end of the stylet (90) being fixed to an inner surface of the ring.

2. The balloon catheter (10) as claimed in any previous claim, further comprising a removable gas insert (98) disposed within at least a portion of the tube (56).

3. The balloon catheter (10) as claimed in any previous claim, wherein the tip (20) is J-shaped.

4. The balloon catheter (10) as claimed in any previous claim, wherein the tip (20) has a fiberoptic sensor (22) fixed to it.

5. The balloon catheter (10) as claimed in any previous claim, wherein the tip (20) has a fiberoptic sensor (22) fixed to it and a fiberoptic fiber (24) extending from the sensor through a space defined by the balloon membrane (30) and through a gas shuttle lumen defined by the tube (56), said fiber being secured to the stylet (90).

6. The balloon catheter (10) as claimed in claim 1, wherein the tip (20) has a fiberoptic sensor (22) fixed to it and a fiberoptic fiber (24) extending from the sensor through a space defined by the balloon membrane (30) and through a gas shuttle lumen defined by the tube (56), said fiber being sandwiched between the stylet (90) and a thin walled tube disposed over the stylet.

7. The balloon catheter (10) as claimed in claim 1, wherein the tip (20) has a fiberoptic sensor (22) fixed to it and a fiberoptic fiber (24) extending from the sensor through a space defined by the balloon membrane (30) and through a gas shuttle lumen defined by the tube (56), said fiber being embedded in the stylet (90).

8. The balloon catheter (10) as claimed in claim 1, wherein the tip (20) has a fiberoptic sensor (22) fixed to it and a fiberoptic fiber (24) extending from the sensor through a space defined by the balloon membrane (30) and through a gas shuttle lumen defined by the tube (56), said stylet (90) comprising a tube and said fiber passing through said tube.

9. The balloon catheter (10) as claimed in claim 1 wherein the tip (20) comprises an inner surface and a pocket (82), and wherein a fiberoptic sensor (22) fixed to the tip has a pressure sensing surface (80), the fiberoptic sensor is embedded in the tip such that the pressure sensing surface is exposed to said pocket.

10. The balloon catheter (10) as claimed in claim 1, wherein the tip (20) comprises an inner surface, a distal sloping portion, and a pocket (82), and wherein a fiberoptic sensor (22) fixed to the tip has a pressure sensing surface (80), the fiberoptic sensor is embedded in the tip such that the pressure sensing surface is exposed to said pocket, the pocket extends from the distal sloping portion to a point between said distal sloping portion and a proximal end of the tip.

11. The balloon catheter (10) as claimed in claim 1, wherein the tip (22) comprises an inner surface and a pocket (82) filled with a protective material, and wherein a fiberoptic sensor (22) fixed to the tip has a pressure sensing surface (80), the fiberoptic sensor is embedded in the tip such that the pressure sensing surface is exposed to said pocket.

12. The balloon catheter (10) as claimed in claim 1, wherein the tip (20) comprises an inner surface and a pocket (82) filled with a gel, and wherein a fiberoptic sensor (22) fixed to the tip has a pressure sensing surface (80), the fiberoptic sensor is embedded in the tip such that the pressure sensing surface is exposed to said pocket.

13. The balloon catheter (10) as claimed in claim 1, wherein the tip (20) comprises an inner surface and a pocket (82), and wherein a fiberoptic sensor (22) fixed to the tip has a pressure sensing surface (80), the fiberoptic sensor is embedded in the tip such that the pressure sensing surface is exposed to said pocket, said pocket is sealed by a membrane (84).

14. The balloon catheter (10) as claimed in claim 1, wherein the tip (20) comprises an inner surface and a pocket (82) filled with a protective material, and wherein a fiberoptic sensor (22) fixed to the tip has a pressure sensing surface (80), the fiberoptic sensor is embedded in the tip such that the pressure sensing surface is exposed to said pocket, said pocket is sealed by the balloon membrane (30).

## Patentansprüche

1. Ballonkatheter (10), welcher eine Ballonmembran (30), einen Schlauch (56), eine Spitze (20) und einen Führungsstab (90) umfasst, wobei ein distales Ende des Schlauchs mit einem proximalen Ende der Ballonmembran verbunden ist, die Spitze mit einem distalen Ende der Ballonmembran verbunden ist, ein distales Ende des Führungsstabs mit der Spitze verbunden ist und ein proximales Ende des Führungsstabs mit einem distalen Ende des Schlauchs verbunden ist, wobei der Führungsstab in Richtung des distalen Endes biegsamer ist als in Richtung des proximalen Endes, wobei ein Verstärkungsring (91) innerhalb eines distalen Endes des Schlauchs (56) angeordnet ist, **dadurch gekennzeichnet, dass** ein proximales Ende des Führungsstabs (90) an einer inneren Fläche des Rings befestigt ist.

2. Ballonkatheter (10) nach einem vorhergehenden Anspruch, welcher ferner einen abnehmbaren Gaseinlass (98) umfasst, der zumindest innerhalb eines Abschnitts des Schlauchs (56) angeordnet ist.

3. Ballonkatheter (10) nach einem der vorhergehenden Ansprüche, wobei die Spitze (20) eine J-Form aufweist.

4. Ballonkatheter (10) nach einem der vorhergehenden Ansprüche, wobei die Spitze (20) einen Glasfasersensor (22) aufweist, der an ihr befestigt ist.

5. Ballonkatheter (10) nach einem der vorhergehenden Ansprüche, wobei die Spitze (20) einen Glasfasersensor (22), der an ihr befestigt ist, und eine Glasfaser (24) aufweist, die sich von dem Sensor ausgehend durch einen Raum, der durch die Ballonmembran (30) definiert ist, und durch ein durch den Schlauch (56) definiertes, in zwei Richtungen durchströmbares Gaslumen erstreckt, wobei die Faser an dem Führungsstab (90) befestigt ist.

6. Ballonkatheter (10) nach Anspruch 1, wobei die Spitze (20) einen Glasfasersensor (22), der an ihr befestigt ist, und eine Glasfaser (24) aufweist, die sich von dem Sensor ausgehend durch einen Raum, der durch die Ballonmembran (30) definiert ist, und durch ein durch den Schlauch (56) definiertes, in zwei Richtungen durchströmbares Gaslumen erstreckt, wobei die Faser zwischen dem Führungsstab (90) und einem dünnwandigen Schlauch eingebettet ist, der über dem Führungsstab angeordnet ist.

7. Ballonkatheter (10) nach Anspruch 1, wobei die Spitze (20) einen Glasfasersensor (22), der an ihr befestigt ist, und eine Glasfaser (24) aufweist, die sich von dem Sensor ausgehend durch einen Raum, der durch die Ballonmembran (30) definiert ist, und durch ein durch den Schlauch (56) definiertes, in zwei Richtungen durchströmbares Gaslumen erstreckt, wobei die Faser in dem Führungsstab (90) eingebettet ist.

8. Ballonkatheter (10) nach Anspruch 1, wobei die Spitze (20) einen Glasfasersensor (22), der an ihr befestigt ist, und eine Glasfaser (24) aufweist, die sich von dem Sensor ausgehend durch einen Raum, der durch die Ballonmembran (30) definiert ist, und durch ein durch den Schlauch (56) definiertes, in zwei Richtungen durchströmbares Gaslumen erstreckt, wobei der Führungsstab (90) einen Schlauch umfasst und die Faser durch den Schlauch hindurch verläuft.

9. Ballonkatheter (10) nach Anspruch 1, wobei die Spitze (20) eine innere Fläche und eine Tasche (82) umfasst, und wobei ein Glasfasersensor (22), der an der Spitze befestigt ist, eine druckempfindliche Fläche (80) aufweist, wobei der Glasfasersensor derart in die Spitze eingebettet ist, dass die druckempfindliche Fläche zu der Tasche hin ausgerichtet ist.

10. Ballonkatheter (10) nach Anspruch 1, wobei die Spitze (20) eine innere Fläche, einen distalen schrägen Abschnitt und eine Tasche (82) umfasst, und wobei ein Glasfasersensor (22), der an der Spitze befestigt ist, eine druckempfindliche Fläche (80) aufweist, wobei der Glasfasersensor derart in die Spitze eingebettet ist, dass die druckempfindliche Fläche zu der Tasche hin ausgerichtet ist, wobei sich die Tasche von dem distalen schrägen Abschnitt zu einem Punkt zwischen dem distalen schrägen Abschnitt und einem proximalen Ende der Spitze erstreckt.

11. Ballonkatheter (10) nach Anspruch 1, wobei die Spitze (20) eine innere Fläche und eine Tasche (82) umfasst, die mit einem schützenden Material gefüllt ist, und wobei ein Glasfasersensor (22), der an der Spitze befestigt ist, eine druckempfindliche Fläche (80) aufweist, wobei der Glasfasersensor derart in die Spitze eingebettet ist, dass die druckempfindliche Fläche zu der Tasche hin ausgerichtet ist.

12. Ballonkatheter (10) nach Anspruch 1, wobei die Spitze (20) eine innere Fläche und eine Tasche (82) umfasst, die mit einem Gel gefüllt ist, und wobei ein Glasfasersensor (22), der an der Spitze befestigt ist, eine druckempfindliche Fläche (80) aufweist, wobei der Glasfasersensor derart in die Spitze eingebettet ist, dass die druckempfindliche Fläche zu der Tasche hin ausgerichtet ist.

13. Ballonkatheter (10) nach Anspruch 1, wobei die Spitze (20) eine innere Fläche und eine Tasche (82) umfasst, und wobei ein Glasfasersensor (22), der an der Spitze befestigt ist, eine druckempfindliche Fläche (80) aufweist, wobei der Glasfasersensor derart in die Spitze eingebettet ist, dass die druckempfindliche Fläche zu der Tasche hin ausgerichtet ist, wobei die Tasche durch eine Membran (84) versiegelt ist.

14. Ballonkatheter (10) nach Anspruch 1, wobei die Spitze (20) eine innere Fläche und eine Tasche (82) umfasst, die mit einem schützenden Material gefüllt ist, und wobei ein Glasfasersensor (22), der an der Spitze befestigt ist, eine druckempfindliche Fläche (80) aufweist, wobei der Glasfasersensor derart in die Spitze eingebettet ist, dass die druckempfindliche Fläche zu der Tasche hin ausgerichtet ist, wobei die Tasche durch die Ballonmembran (30) versiegelt ist.

## Revendications

1. Cathéter à ballonnet (10) comprenant une membrane de ballonnet (30), un tube (56), un embout (20) et un stylet (90), une extrémité distale du tube est raccordée à une extrémité proximale de la membrane de ballonnet, l'embout est raccordé à une extrémité distale de la membrane de ballonnet, une extrémité distale du stylet est raccordée à l'embout, une extrémité proximale du stylet est raccordée à une extrémité distale du tube, le stylet étant plus souple vers l'extrémité distale que vers l'extrémité proximale, dans lequel un anneau de renfort (91) est disposé dans une extrémité distale du tube (56), **caractérisé en ce qu'**une extrémité proximale du stylet (90) est fixée à une surface interne de l'anneau.

2. Cathéter à ballonnet (10) tel que revendiqué dans une quelconque revendication précédente, comprenant en outre un insert à gaz amovible (98) disposé dans au moins une partie du tube (56).

3. Cathéter à ballonnet (10) tel que revendiqué dans une quelconque revendication précédente, dans lequel l'embout (20) est en forme de J.

4. Cathéter à ballonnet (10) tel que revendiqué dans une quelconque revendication précédente, dans lequel l'embout (20) a un détecteur à fibre optique (22) fixé à celui-ci.

5. Cathéter à ballonnet (10) tel que revendiqué dans une quelconque revendication précédente, dans lequel l'embout (20) a un détecteur à fibre optique (22) fixé à celui-ci et une fibre optique (24) s'étendant du détecteur à travers un espace défini par la membrane de ballonnet (30) et à travers un lumen de transport de gaz défini par le tube (56), ladite fibre étant fixé au stylet (90).

6. Cathéter à ballonnet (10) tel que revendiqué dans la revendication 1, dans lequel l'embout (20) a un détecteur à fibre optique (22) fixé à celui-ci et une fibre optique (24) s'étendant du détecteur à travers un espace défini par la membrane de ballonnet (30) et à travers un lumen de transport de gaz défini par le tube (56), ladite fibre étant intercalée entre le stylet (90) et un tube à parois minces disposé sur le stylet.

7. Cathéter à ballonnet (10) tel que revendiqué dans la revendication 1, dans lequel l'embout (20) a un détecteur à fibre optique (22) fixé à celui-ci et une fibre optique (24) s'étendant du détecteur à travers un espace défini par la membrane de ballonnet (30) et à travers un lumen de transport de gaz défini par le tube (56), ladite fibre étant incorporée dans le stylet (90).

8. Cathéter à ballonnet (10) tel que revendiqué dans la revendication 1, dans lequel l'embout (20) a un détecteur à fibre optique (22) fixé à celui-ci et une fibre optique (24) s'étendant du détecteur à travers un espace défini par la membrane de ballonnet (30) et à travers un lumen de transport de gaz défini par le tube (56), ledit stylet (90) comprenant un tube et ladite fibre passant à travers le tube.

9. Cathéter à ballonnet (10) tel que revendiqué dans la revendication 1, dans lequel l'embout (20) comprend une surface interne et une poche (82), et dans lequel un détecteur à fibre optique (22) fixé à l'embout a une surface de détection de pression (80), le capteur à fibre optique est incorporé dans l'embout de telle sorte que la surface de détection de pression est exposée à ladite poche.

10. Cathéter à ballonnet (10) tel que revendiqué dans la revendication 1, dans lequel l'embout (20) comprend une surface interne, une partie distale en pente et une poche (82), et dans lequel un détecteur à fibre optique (22) fixé à l'embout a une surface de détection de pression (80), le détecteur à fibre optique est incorporé dans l'embout de telle sorte que la surface de détection de pression est exposée à ladite poche, la poche s'étend de la partie distale en pente à un point situé entre ladite partie distale en pente et une extrémité proximale de l'embout.

11. Cathéter à ballonnet (10) tel que revendiqué dans la revendication 1, dans lequel l'embout (20) comprend une surface interne et une poche (82) remplie d'un matériau protecteur, et dans lequel un détecteur à fibre optique (22) fixé à l'embout a une surface de détection de pression (80), le détecteur à fibre optique est incorporé dans l'embout de telle sorte que la surface de détection de pression est exposée à ladite poche.

12. Cathéter à ballonnet (10) tel que revendiqué dans la revendication 1, dans lequel l'embout (20) comprend une surface interne et une poche (82) remplie d'un gel, et dans lequel un détecteur à fibre optique (22) fixé à l'embout a une surface de détection de pression (80), le détecteur à fibre optique est incorporé dans l'embout de telle sorte que la surface de détection de pression est exposée à ladite poche.

13. Cathéter à ballonnet (10) tel que revendiqué dans la revendication 1, dans lequel l'embout (20) comprend une surface interne et une poche (82), et dans lequel un détecteur à fibre optique (22) fixé à l'embout a une surface de détection de pression (80), le détecteur à fibre optique est incorporé dans l'embout de telle sorte que la surface de détection de pression est exposée à ladite poche, ladite poche est scellée par une membrane (84).

14. Cathéter à ballonnet (10) tel que revendiqué dans la revendication 1, dans lequel l'embout (20) comprend une surface interne et une poche (82) remplie d'un matériau protecteur, et dans lequel un détecteur à fibre optique (22) fixé à l'embout a une surface de détection de pression (80), le détecteur à fibre optique est incorporé dans l'embout de telle sorte que la surface de détection de pression est exposée à ladite poche, ladite poche est scellée par la membrane de ballonnet (30).
